# EUROPEAN PATENT SPECIFICATION

(11) **EP 2 994 120 B1**
(45) Date of publication and mention of the grant of the patent: **09.10.2019**
(21) Application number: 14729541.4
(22) Date of filing: 05.05.2014
(51) Int. Cl.: A61K 31/137, A61K 31/4164, A61K 31/4174, A61K 31/498, A61K 31/165, A61K 9/00, A61P 17/02

(54) **ALPHA ADRENERGIC AGONISTS FOR THE TREATMENT OF TISSUE TRAUMA**
ALPHA-ADRENERGE AGONISTEN ZUR BEHANDLUNG VON GEWEBETRAUMA
AGONISTES ALPHA-ADRÉNERGIQUES POUR LE TRAITEMENT D'UN TRAUMATISME TISSULAIRE

(30) Priority: 06.05.2013 US 201361819927 P
(43) Date of publication of application: 16.03.2016
(73) Proprietor: ALLERGAN, INC., Irvine, CA 92612 (US)
(72) Inventor: GIL, Daniel W., Corona Del Mar, California 92625 (US); ANDREWS-JONES, Lydia L., Lake Forest, California 92630 (US); HSIA, Edward C., Irvine, California 92620 (US); TIAN, Mingting, Irvine, California 92602 (US)
(74) Representative: Hoffmann Eitle
(86) International application number: PCT/US2014/036778
(87) International publication number: WO 2014/182610

(56) References cited:
- EP-A1- 2 329 849
- WO-A1-97/04764
- WO-A1-2012/118704
- WO-A1-2013/001073
- WO-A1-2013/010032
- WO-A1-2013/070861
- WO-A2-2005/115395
- WO-A2-2009/061431
- FR-A1- 2 966 365
- US-A- 3 536 730
- TURSS R ET AL: "Corneal nutrition in alkali burns and the effect of tolazoline", ALBRECHT VON GRAEFES ARCHIV FUER KLINISCHE UND EXPERIMENTELLE OPHTHALMOLOGIE - ALBRECHT VON GRAEFE'S ARCHIVE FOR CLINICAL AND EXPERIMENTAL OPHTHALMOLOGY, SPRINGER, BERLIN, DE, vol. 190, no. 2, 1 January 1974 (1974-01-01), pages 155-163, XP009179373, ISSN: 0065-6100
- SUSUMU OKABE ET AL: "EFFECTS OF ADRENERGIC BLOCKING AGENTS ON GASTRIC SECRETION AND STRESS-INDUCED GASTRIC ULCER IN RATS", THE JAPANESE JOURNAL OF PHARMACOLOGY, vol. 20, no. 1, 1 January 1970 (1970-01-01), pages 10-15, XP055131729, ISSN: 0021-5198, DOI: 10.1254/jjp.20.10
- M Tian ET AL: "Photobiology | ABSTRACTS 1251 The alpha adrenergic receptor agonist oxymetazoline decreases erythema and inflammation in a UVB-induced sunburn model", Journal of investigative dermatology, 1 May 2013 (2013-05-01), page S213, XP055131572, Retrieved from the Internet: URL:http://www.nature.com/jid/journal/v133 /n1s/pdf/jid2013104a.pdf [retrieved on 2014-07-24]

## Description

This application claims the benefit of U.S. Application Serial No. 61/819,927, filed on May 6, 2013.

### FIELD OF THE INVENTION

The present invention is directed to a composition for use in a method of treating tissue trauma (such as damage from solar or ultraviolet radiation, wounds, and burns) by administration of an alpha adrenergic agonist (such as oxymetazoline hydrochloride) as defined in the claims.

### BACKGROUND OF THE INVENTION

It has been recognized that alpha-adrenergic agonists are useful for treating or preventing skin redness in conditions including rosacea, acne, acute sunburn and chronic sun damage. The effectiveness of alpha-adrenergic agonists is due to their action on vascular smooth muscle that results in constriction of cutaneous blood vessels. The sympathetic nervous system mediates peripheral vasoconstriction via activation of both α1- and α2-adrenergic receptors on vascular smooth muscle. Knockout mice studies have demonstrated that either the α2A- or the α2C-adrenergic receptors mediate cutaneous vasoconstriction in the tail, depending on the air temperature (Chotani, Flavahan et al. 2000), and that α1-adrenergic receptors also play an important role in peripheral vasoconstriction (Duka, Gavras et al. 2000). In human subjects with reduced sympathetic activity, the mixed α2/α1 agonist clonidine or the α2-selective agonist dexmedetomidine reduced finger blood flow (Talke, Lobo et al. 2001; Talke, Lobo et al. 2003).

### References:

Chotani, M. A., S. Flavahan, et al. (2000). "Silent alpha(2C)-adrenergic receptors enable cold-induced vasoconstriction in cutaneous arteries." Am J Physiol Heart Circ Physiol 278(4):H1075-1083.
Duka, I., I. Gavras, et al. (2000). "Role of the postsynaptic alpha(2)-adrenergic receptor subtypes in catecholamine-induced vasoconstriction." Gen Pharmacol 34(2):101-106. Talke, P., E. Lobo, et al. (2003). "Systemically administered alpha2-agonist-induced peripheral vasoconstriction in humans." Anesthesiology 99(1):65-70. Talke, P.O., E. P. Lobo, et al. (2001). "Clonidine-induced vasoconstriction in awake volunteers." Anesth Analg 93(2):271-276, 271st contents page.

### SUMMARY OF THE INVENTION

The present invention provides a composition for use in a method of treating tissue trauma in a subject, comprising topically administering to the tissue area of the subject affected by said trauma a composition comprising a therapeutically effective amount of at least one alpha adrenergic agonist, as defined in the claims.

In another aspect, the disclosure provides a method for treating/alleviating the pain or discomfort associated with aesthetic or plastic surgery or cosmetology procedures in a patient in need thereof, which comprises administering to said patient a pharmaceutical composition comprising a therapeutically effective amount of at least one alpha adrenergic agonist.

### BRIEF DESCRIPTION OF THE DRAWINGS

Figure 1 shows percentage of each group receiving each score for overall healing. Subjects were treated with vehicle or 0.5% oxymetazoline hydrochloride in vehicle at 20 minutes, 4 hours or 8 hours following the trauma.
Figure 2 shows percentage of group receiving each score for epidermal separation. Subjects were treated with vehicle or 0.5% oxymetazoline hydrochloride in vehicle at 20 minutes, 4 hours or 8 hours following the trauma.
Figure 3 shows the percentage of each group receiving each score for epidermal hyperplasia. Subjects were treated with vehicle or 0.5% oxymetazoline hydrochloride in vehicle at 20 minutes, 4 hours or 8 hours following the trauma.
Figure 4 shows the histological sections from vehicle-treated (left) and 8 hr 0.5% oxymetazoline hydrochloride-treated (right) mice; skin collected at 48 hours following injury. ↓ = necrotic epidermis, * shows separation of epidermis with exposed dermis, ↑ = epidermal proliferation beneath necrotic epidermis to cover the defect, ↑↑ = adaptive epidermal hyperplasia.

### DETAILED DESCRIPTION OF THE INVENTION

While investigating the reduction of erythema by the alpha-adrenergic agonist oxymetazoline hydrochloride in a UVB-induced sunburn model, a surprising utility in addition to reduction of erythema was observed. It was surprisingly found that oxymetazoline hydrochloride also promoted skin healing following tissue injury. Exposure of hairless mice to UVB irradiation results in a rapid sunburn-like response characterized by erythema, cutaneous blood vessel dilation, tactile hypersensitivity and inflammation peaking within 2-4 hr. The burn is severe enough to result in subsequent tissue damage characterized by epidermal necrosis with separation of the epidermis from the dermis and reactive changes such as adaptive epidermal hyperplasia at 48 hr.

Accordingly, the present invention provides a composition for use in a method of treating tissue trauma in a subject, comprising topically administering to the tissue area of the subject affected by said trauma a composition comprising a therapeutically effective amount of at least one alpha adrenergic agonist as defined in the claims.

The tissue trauma is a burn that is severe enough to result in subsequent tissue damage characterized by at least one condition selected from the group consisting of epidermal necrosis, separation of the epidermis from the dermis and adaptive healing response, wherein the adaptive healing response is selected from epidermal proliferation and hyperplasia.

In one embodiment, the treatment of the tissue trauma results in (a) the reduction of severity, (b) prevention of development, or (c) more rapid reepithelialization and closure of open wounds, blisters, excoriations, erosions or ulcers.

In another embodiment, the treatment of tissue trauma results in the enhancement of the healing of the tissue trauma.

In another embodiment, the treatment of the tissue trauma results in the prevention or minimized formation of fine line scars, hypertrophic scars and keloids.

In another embodiment, the tissue area is selected from the group consisting of corneal epithelium, skin, and mucous membranes (such as mouth, those in the gastrointestinal tract, and rectal and vaginal mucosa).

As set forth above, in another aspect, the present disclosure provides a method for alleviating the pain or discomfort associated with aesthetic or plastic surgery or cosmetology procedures in a patient in need thereof, which comprises treating said patient with a pharmaceutical composition comprising a therapeutically effective amount of at least one alpha adrenergic agonist. Such procedures include dermal filler injections, neurotoxin injections, Botulinum toxin injections, laser procedures, breast augmentations, breast lifts, breast reductions, face lifts, tummy tucks.

In another embodiment, the alpha adrenergic agonist may be administered in combination with an anesthetic.

The alpha adrenergic agonist of the present invention comprises a compound with an imidazoline structure.

The compound with the imidazoline structure is selected from the group consisting of oxymetazoline, xylometazoline, naphazoline, mivazerol and dexmedetomidine; or a pharmaceutically acceptable salt thereof.

In an especially preferred embodiment, the alpha adrenergic agonist of the present invention is oxymetazoline hydrochloride, having the structure:

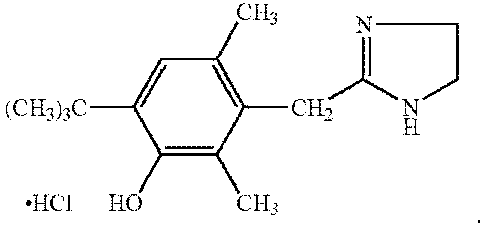

The alpha adrenoceptor agonist of the present invention can be used in amounts of about 0.01% up to about 30%, and in one embodiment about 0.001% up to about 3%, and in one embodiment from about 0.01% up to about 30%, and in one embodiment from about 0.1% up to about 10% by weight based on the total weight of the composition. One alpha agonist or a combination or a combination of two or more agonists can be used. The alpha agonists can be used as the sole active agent, or the composition can comprising additional active agents having different functions as set forth below.

The composition of the present invention can further comprise a pharmaceutically/dermatologically acceptable carrier or vehicle. The term "pharmacologically/dermatologically acceptable carriers", as used herein, means that the carrier is suitable for topical application to tissue area, has good aesthetic properties, is compatible with the active agents of the present invention and any other components, and will not cause any untoward safety or toxicity concerns. The carrier can be in a wide variety of forms. For example, emulsion carriers, including, but not limited to, oil-in-water, water-in-oil, water-in-oil-in-water, and oil-in-water-in-silicon emulsions, are useful herein. As will be understood by the skilled artisan, a given component will distribute primarily into either the water or oil/silicon phase, depending on the water solubility/dispersibility of the component in the composition. A safe and effective amount of carrier is from about 50% to about 99.999%, and in one embodiment from about 70% to about 99.99%.

The carrier or vehicle of the invention will have dramatic effects on the concentrations of the active ingredients selected. The preferred embodiments employ active ingredients in amounts effective to achieve clinical efficacy without causing systemic side effects.

The compositions according to the invention may comprise all pharmaceutical forms normally utilized for the topical route of administration and known to practitioners of this art including solutions, gels, lotions creams, ointments, foams, mousses, emulsions, microemulsions, milks, serums, aerosols, sprays, dispersions, microcapsules, vesicles and microparticles thereof. The subject compositions may also be formulated as solid preparations constituting soaps or cleansing bars. These compositions are formulated according to conventional techniques.

In another embodiment, the affected tissue area is the skin, and the topical administration involves rubbing the composition onto the tissue area, applying the composition to the tissue area through a dermal patch, or injecting the composition into the epidermis via a micro-injector.

The composition, if desired, can contain various known bases such as excipients, binders, lubricants, and disintegrants. If desired, it can also contain oily materials such as various fats, oils, waxes, hydrocarbons, fatty acids, higher alcohols, ester oils, metallic soaps, animal or vegetable extracts, hydrophilic or lipophilic gelling agents, hydrophilic or lipophilic active agents, pharmaceutically effective components such as vitamins, hormones, amino acids, surfactants, colorants, dyes, pigments, fragrances, odor absorbers, antiseptics, preservatives, bactericides, humectants, thickeners, solvents, fillers, antioxidants, sequestering agents, sunscreens, or any other known components and additives as long as the effects of the present invention are not impaired.

Examples of suitable oils includes mineral oils, plant oils such as peanut oil, sesame oil, soybean oil, safflower oil, sunflower oil, animal oils such as lanolin or perhydrosqualene, synthetic oils such as purcellin oil, silicone oils such as cyclomethicome among others. Fatty alcohols, fatty acids such as stearic acid and waxes such as paraffin wax, carnauba wax or beeswax may also be used as fats. The composition may also contain emulsifying agents such as glyceryl stearate, solvents such as lower alcohols including ethanol, isopropanol, and propylene glycol, hydrophilic gelling agents including carboxyvinyl polymers or acrylic copolymers, polyacrylamides, polysaccharides, lipophilic gelling agents or fatty acid metal salts among others, hydrophilic acting agents such as amino acids, sugars, starch or urea, lipophilic active agents such as retinol or tocopherol.

In another embodiment, the composition of the present invention further comprises at least one ingredient selected from the group consisting of water, a solvent, a preservative, a surfactant, a gelling agent, and a pH balancer.

The compositions may contain antimicrobial preservatives in some embodiments. In several embodiments, antimicrobial preservatives include, but are not limited to, methylparaben, propylparaben, benzyl alcohol, ethylhexylglycerin, potassium sorbate, phenoxyethanol, EDTA, grapefruit seed extract, tea tree oil, sodium benzoate, dehydroacetic acid, and combinations thereof. In some embodiments, anti-fungal preservatives are used alone or in combination with anti-bacterial preservatives.

Suitable gelling agents known in the art, including those used in the two-phase or single-phase gel systems, can be used in the present invention. Some examples of suitable gelling agents are disclosed in REMINGTON: THE SCIENCE AND PRACTICE OF PHARMACY 1517-1518 (Alfonso R. Gennaro ed. 19th ed. 1995). The gelling agents used in embodiments of the present invention, include, but are not limited to, one or more hydrophilic and hydroalcoholic gelling agents used in the cosmetic and pharmaceutical industries. Suitable hydroalcoholic gelling agents include "CARBOPOL®" (B.F. Goodrich, Cleveland, Ohio), "HYPAN®" (Kingston Technologies, Dayton, N.J.), "NATROSOL®" (Aqualon, Wilmington, Del.), "KLUCEL®" (Aqualon, Wilmington, Del.), or "STABlLEZE®" (ISP Technologies, Wayne, N.J.).

### Assay/Method

A xenon arc lamp (Newport Corp, USA) was used as a source of UVB. SKH1 hairless mice, lying on their stomachs with their left sides covered, were exposed to 120 mJ/cm2 UVB radiation. Regions of back skin were treated topically with oxymetazoline hydrochloride 0.5% gel at 20 min, 4 hr or 8 hr post-UVB. An additional group had the vehicle applied topically at 20 min post UVB exposure. Erythema on the exposed and unexposed back was measured using a chromameter (Konica Minolta) at 2 and 4 hr post-UVB exposure. Skin samples were collected 48 hr following UVB exposure and examined histologically to assess the extent of injury.

### Results

UVB exposure resulted in a 28% increase in erythema that peaked by 2 hr post-UVB exposure. Application of 0.5% oxymetazoline 20 minutes following UVB exposure to the back skin almost completely inhibited the erythema (p<0.001) at the 2 hr timepoint, but there was no inhibition of the erythema at 4 hr post-UVB exposure.

Histological examination at 48 hr showed that application of 0.5% oxymetazoline 20 minutes following UVB exposure to the back skin slightly improved the outcome of healing compared to concurrent vehicle controls, but healing was within the range of historical vehicle controls; therefore the significance of this slight improvement with application at 20 minutes was unknown.

Application of 0.5% oxymetazoline 4 or 8 hours following UVB exposure to the back skin improved the outcome of healing compared to both concurrent vehicle controls, and historical vehicle controls. Improved healing consisted of a shift toward adaptive epidermal hyperplasia and a reduction of epidermal necrosis, and less separation of the epidermis from underlying dermis.

The overall epidermal healing was fair to good in 33% of mice treated with 0.5% oxymetazoline at 20 minutes, 50% of mice treated at 4 hours, and 67% of mice treated at 8 hours post UVB exposure, as compared to 0% of vehicle treated mice. Therefore this study shows that treatment with 0.5% oxymetazoline topically 4-8 hours following UVB exposure improves the healing of UVB exposed epidermis. Since oxymetazoline has a very strong effect on erythema, but not on healing, in the first 2 hrs post-UVB exposure, the data also demonstrate that the enhanced healing stimulated by oxymetazoline is distinct from its vasoconstrictive actions to reduce erythema.

### Examples

In order to further illustrate the present invention and the advantages thereof, the following specific examples are given, it being understood that the same are illustrative and in no ways limitative.

### Example 1

The following formulation of oxymetazoline was used:

| **Ingredient** | **Concentration % w/w** |
|---|---|
| Oxymetazoline hydrochloride | 0.5% |
| Ethanol | 10 |
| Transcutol | 10 |
| Carbopol 980 | 0.35 |
| Triethanolamine | 0.525 |
| Glycerin | 2.0 |
| Purified water | Q.s. |

The vehicle used for the purposes of testing the formulation comprises all of the above ingredients in the above table (with the same relative amounts of each, made up to 100% with purified water) with the exception of oxymetazoline hydrochloride.

### Example 2

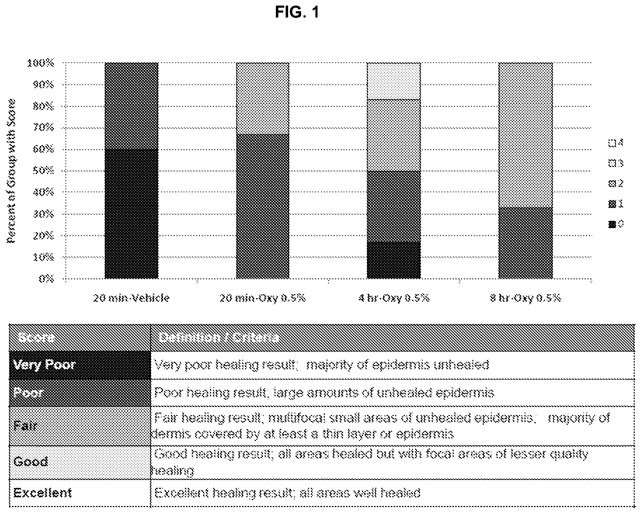

Figure 1 shows the percentage of each group receiving each score for overall healing. Subjects were treated with vehicle or 0.5% oxymetazoline hydrochloride in vehicle at 20 minutes, 4 hours or 8 hours following the trauma.

One of many functions of the skin is to provide a protective barrier for underlying soft tissues. When the skin is injured and is no longer providing this function, this can result in many detrimental sequelae, such as dehydration, and introduction of infection. Therefore, when the skin is injured, it must adapt quickly and heal itself, through many interacting physiologic processes. In this study, the overall healing score given 48 hours following the injury was a measure of the overall effectiveness of this physiologic response to the UVB injury. The application of 0.5% oxymetazoline hydrochloride at 4 or 8 hours post UVB exposure resulted in a more positive outcome as seen at 48 hours following injury for many individuals; application of 0.5% oxymetazoline hydrochloride at 20 minutes post UVB exposure potentially improved the outcome at 48 hours for a few individuals. Group size: n= 5 for vehicle group, and n=6 for all 0.5% Oxymetazoline groups.

### Example 3

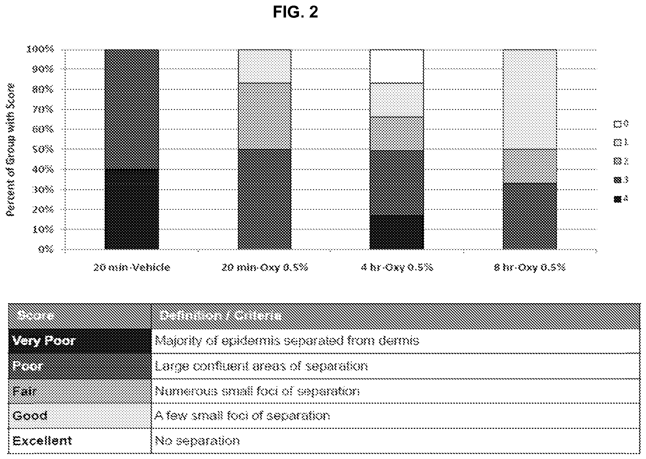

Figure 2 shows the percentage of each group receiving each score for epidermal separation. Subjects were treated with vehicle or 0.5% oxymetazoline hydrochloride in vehicle at 20 minutes, 4 hours or 8 hours following the trauma.

The epidermis is the outermost layer of the skin in contact with the environment and crucial to the overall health and barrier function of the skin. When the epidermis becomes necrotic, it can begin to separate from the underlying dermis, and thus its function has been lost. An adequate physiologic response to injury should either prevent epidermal necrosis in the first place, and/or to promote rapid epidermal proliferation to cover any defects that may result. The epidermal separation score given 48 hours following injury indicated how much dermis was exposed as a result of failure of the skin to either prevent epidermal loss, and/or to replace lost epidermis via epidermal proliferation. The application of 0.5% oxymetazoline hydrochloride at 20 minutes, 4 hours, or 8 hours post UVB exposure, reduced the occurrence and/or severity of epidermal separation seen at 48 hours following UVB exposure for most individuals. Group size: n= 5 for vehicle group, and n=6 for all 0.5% oxymetazoline hydrochloride groups.

### Example 4

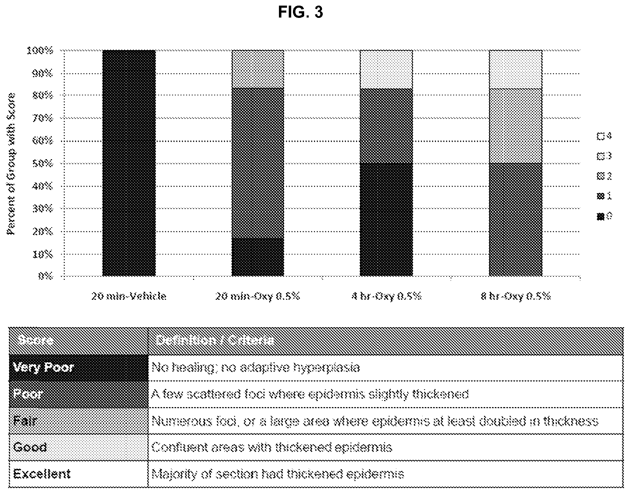

Figure 3 shows the percentage of each group receiving each score for epidermal hyperplasia. Subjects were treated with vehicle or 0.5% oxymetazoline hydrochloride. in vehicle at 20 minutes, 4 hours or 8 hours following the trauma.

Following an injury to skin severe enough to potentially result in epidermal loss, the healing epidermis begins to proliferate and first spreads out in a thin attenuated layer to cover any defects, then becomes hyperplastic as healing progresses. Thus in this case, 48 hours following the injury, the presence of epidermal hyperplasia indicated more advanced and effective progression of the adaptive healing response. The application of 0.5% oxymetazoline hydrochloride, especially when applied 8 hours following the injury, resulted in more individuals who had progressed to advanced healing by 48 hours post injury. Group size: n= 5 for vehicle group, and n=6 for all 0.5% oxymetazoline hydrochloride groups.

### Example 5

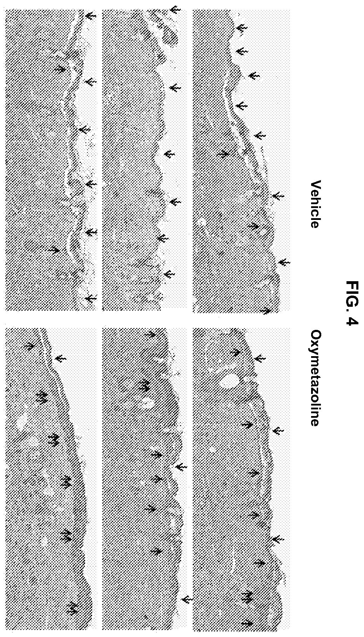

Figure 4 shows the histological sections from vehicle-treated (left) and 8 hr 0.5% oxymetazoline hydrochloride-treated (right) mice; skin collected at 48 hours following injury. ↓ = necrotic epidermis, * shows separation of epidermis with exposed dermis, ↑ = epidermal proliferation beneath necrotic epidermis to cover the defect, ↑↑ = adaptive epidermal hyperplasia. In skin that was treated only with vehicle, this degree of UVB exposure had been too great an injury for the skin to respond adequately, resulting in epidermal failure by 48 hours following injury in all subjects, with exposure of the underlying dermis due to epidermal necrosis (↓) and separation (*). In contrast, the skin treated with 0.5% oxymetazoline hydrochloride 8 hours following injury showed a greater degree of a positive adaptive response by 48 hours following the injury, with more epidermal proliferation (↑) covering any defects, and even well progressed adaptive hyperplasia (↑↑).

The foregoing descriptions details specific methods and compositions that can be employed to practice the present invention, and represents the best mode contemplated. It should not be construed as limiting the overall scope hereof; rather, the ambit of the present invention is to be governed only by the lawful construction of the appended claims.

## Claims

1. A composition comprising a therapeutically effective amount of at least one alpha adrenergic agonist, for use in a method of treating tissue trauma in a subject, the method comprising topically administering said composition to the tissue area of the subject affected by said trauma , wherein the tissue trauma is a burn that is severe enough to result in subsequent tissue damage **characterized by** at least one condition selected from the group consisting of epidermal necrosis, separation of the epidermis from the dermis and adaptive healing response, wherein the adaptive healing response is selected from epidermal proliferation and hyperplasia, wherein the alpha adrenergic agonist comprises a compound with an imidazoline structure, wherein the compound with the imidazoline structure is selected from the group consisting of oxymetazoline, xylometazoline, naphazoline, mivazerol and dexmedetomidine; or a pharmaceutically acceptable salt thereof.

2. Composition for use of claim 1, wherein the burn is caused by a chemical, heat, or solar radiation.

3. Composition for use of claim 1, wherein the treatment results in (a) the reduction of severity, (b) prevention of development, or (c) more rapid reepithelialization and closure of open wounds, blisters, excoriations, erosions or ulcers.

4. Composition for use of claim 1, wherein the treatment results in the prevention or minimized formation of fine line scars, hypertrophic scars and keloids.

5. Composition for use of claim 1, wherein the treatment results in the enhancement of the healing of the tissue trauma.

6. Composition for use of claim 1, wherein the tissue area is selected from the group consisting of corneal epithelium, skin, and mucous membranes.

7. Composition for use of claim 1, wherein the compound is oxymetazoline hydrochloride.

8. Composition for use of claim 1, wherein the composition is selected from the group consisting of solutions, gels, lotions, creams, ointments, foams, emulsions, microemulsions, milks, serums, aerosols, sprays, dispersions, microcapsules, vesicles and microparticles thereof.

9. Composition for use of claim 1, wherein the tissue area is skin, and the topical administration involves rubbing the composition onto the tissue area, applying the composition to the tissue area through a dermal patch, or injecting the composition into the epidermis via a micro-injector.

10. Composition for use of claim 1, wherein the composition comprises about 0.01% to about 30% of the alpha adrenergic agonist.

11. Composition for use of claim 1, wherein the composition further comprises about 50% to about 99.999% of a pharmaceutically acceptable carrier.

12. Composition for use of claim 1, wherein the alpha adrenergic agonist is the sole active agent in the composition.

13. Composition for use of claim 1, wherein the alpha adrenergic agonist is dexmedetomidine.

## Patentansprüche

1. Zusammensetzung, umfassend eine therapeutisch wirksame Menge von mindestens einem alpha-adrenergen Agonisten zur Verwendung in einem Verfahren zur Behandlung eines Gewebetraumas bei einem Subjekt, wobei das Verfahren die topische Verabreichung der Zusammensetzung an den Gewebebereich des Subjekts umfasst, der von dem Trauma betroffen ist, wobei das Gewebetrauma eine Verbrennung ist, die schwer genug ist, um zu einem nachfolgenden Gewebeschaden zu führen, der durch mindestens einen Zustand gekennzeichnet ist, der aus der Gruppe ausgewählt ist, die aus epidermaler Nekrose, Trennung der Epidermis von der Dermis und adaptiver Heilungsreaktion besteht, wobei die adaptive Heilungsreaktion aus epidermaler Proliferation und Hyperplasie ausgewählt ist, wobei der alpha-adrenerge Agonist eine Verbindung mit einer Imidazolinstruktur, wobei die Verbindung mit der Imidazolinstruktur aus der Gruppe ausgewählt ist, die aus Oxymetazolin, Xylometazolin, Naphazolin, Mivazerol und Dexmedetomidin besteht; oder ein pharmazeutisch verträgliches Salz davon umfasst.

2. Zusammensetzung zur Verwendung gemäß Anspruch 1, wobei die Verbrennung durch eine Chemikalie, Hitze oder Sonnenstrahlung verursacht ist.

3. Zusammensetzung zur Verwendung gemäß Anspruch 1, wobei die Behandlung in (a) einer Verringerung des Schweregrads, (b) einer Prävention der Entwicklung oder (c) einem schnelleren Reepithelisieren und Schließen von offenen Wunden, Blasen, Hautabschürfungen, Erosionen oder Geschwüren führt.

4. Zusammensetzung zur Verwendung gemäß Anspruch 1, wobei die Behandlung zur Prävention oder Minimierung der Bildung von feinen linienförmigen Narben, hypertrophen Narben und Keloiden führt.

5. Zusammensetzung zur Verwendung gemäß Anspruch 1, wobei die Behandlung zu einer Verbesserung der Heilung des Gewebetraumas führt.

6. Zusammensetzung zur Verwendung gemäß Anspruch 1, wobei der Gewebebereich aus der Gruppe ausgewählt ist, die aus Hornhautepithel, Haut und Schleimhäuten besteht.

7. Zusammensetzung zur Verwendung gemäß Anspruch 1, wobei die Verbindung Oxymetazolinhydrochlorid ist.

8. Zusammensetzung zur Verwendung gemäß Anspruch 1, wobei die Zusammensetzung aus der Gruppe ausgewählt ist, die aus Lösungen, Gelen, Lotionen, Cremes, Salben, Schäumen, Emulsionen, Mikroemulsionen, Milchen, Seren, Aerosolen, Sprays, Dispersionen, Mikrokapseln, Vesikeln und Mikropartikeln davon besteht.

9. Zusammensetzung zur Verwendung gemäß Anspruch 1, wobei der Gewebebereich Haut ist und das topische Verabreichen das Reiben der Zusammensetzung auf den Gewebebereich, das Auftragen der Zusammensetzung auf den Gewebebereich durch ein Hautpflaster oder das Injizieren der Zusammensetzung in die Epidermis über einen Mikro-Injektor beinhaltet.

10. Zusammensetzung zur Verwendung gemäß Anspruch 1, wobei die Zusammensetzung etwa 0,01% bis etwa 30% des alpha-adrenergen Agonisten umfasst.

11. Zusammensetzung zur Verwendung gemäß Anspruch 1, wobei die Zusammensetzung weiterhin etwa 50% bis etwa 99,999% eines pharmazeutisch verträglichen Trägers umfasst.

12. Zusammensetzung zur Verwendung gemäß Anspruch 1, wobei der alpha-adrenerge Agonist der einzige Wirkstoff in der Zusammensetzung ist.

13. Zusammensetzung zur Verwendung gemäß Anspruch 1, wobei der alpha-adrenerge Agonist Dexmedetomidin ist.

## Revendications

1. Composition comprenant une quantité thérapeutiquement efficace d'au moins un agoniste alpha-adrénergique, pour une utilisation dans un procédé de traitement de traumatisme tissulaire chez un sujet, le procédé comprenant l'administration topique de ladite composition à la zone tissulaire du sujet affectée par ledit traumatisme, dans laquelle le traumatisme tissulaire est une brûlure qui est suffisamment grave pour donner lieu à une lésion tissulaire ultérieure, **caractérisée par** au moins un état sélectionné dans le groupe constitué par la nécrose épidermique, la séparation de l'épiderme et du derme et la réaction de guérison adaptative, dans laquelle la réaction de guérison adaptative est sélectionnée parmi la prolifération épidermique et l'hyperplasie, dans laquelle l'agoniste alpha-adrénergique comprend un composé avec une structure d'imidazoline, dans laquelle le composé avec la structure d'imidazoline est sélectionné dans le groupe constitué par l'oxymétazoline, la xylométazoline, la naphazoline, le mivazérol et la dexmédétomidine ; ou sel pharmaceutiquement acceptable de cette composition .

2. Composition pour une utilisation selon la revendication 1, dans laquelle la brûlure est causée par un produit chimique, de la chaleur ou un rayonnement solaire.

3. Composition pour une utilisation selon la revendication 1, dans laquelle le traitement donne lieu à (a) la réduction de la gravité, (b) l'empêchement du développement, ou (c) une ré-épithélialisation et une fermeture plus rapides de plaies ouvertes, d'ampoules, d'excoriations, d'érosions ou d'ulcères.

4. Composition pour une utilisation selon la revendication 1, dans laquelle le traitement donne lieu à l'empêchement ou à la formation minimisée de fines cicatrices en ligne, de cicatrices hypertrophiques et de chéloïdes.

5. Composition pour une utilisation selon la revendication 1, dans laquelle le traitement donne lieu à l'amélioration de la guérison du traumatisme tissulaire.

6. Composition pour une utilisation selon la revendication 1, dans laquelle la zone tissulaire est sélectionnée à partir du groupe constitué par l'épithélium cornéen, la peau et les membranes muqueuses.

7. Composition pour une utilisation selon la revendication 1, dans laquelle le composé est le chlorhydrate d'oxymétazoline.

8. Composition pour une utilisation selon la revendication 1, dans laquelle la composition est sélectionnée dans le groupe constitué par des solutions, des gels, des lotions, des crèmes, des pommades, des mousses, des émulsions, des microémulsions, des laits, des sérums, des aérosols, des pulvérisateurs, des dispersions, des microcapsules, des vésicules et leurs microparticules.

9. Composition pour une utilisation selon la revendication 1, dans laquelle la zone tissulaire est la peau et l'administration topique implique de frotter la composition sur la zone tissulaire, d'appliquer la composition dans la zone tissulaire à travers un pansement dermique ou d'injecter la composition dans l'épiderme par l'intermédiaire d'un micro-injecteur.

10. Composition pour une utilisation selon la revendication 1, dans laquelle la composition comprend environ 0,01 % à environ 30 % de l'agoniste alpha-adrénergique.

11. Composition pour une utilisation selon la revendication 1, dans laquelle la composition comprend en outre environ 50% à environ 99,999% d'un support pharmaceutiquement acceptable.

12. Composition pour une utilisation selon la revendication 1, dans laquelle l'agoniste alpha-adrénergique est le seul agent actif dans la composition.

13. Composition pour une utilisation selon la revendication 1, dans laquelle l'agoniste alpha-adrénergique est la dexmédétomidine.
